# NEW EUROPEAN PATENT SPECIFICATION

(11) **EP 2 281 591 B2**
(45) Date of publication and mention of the opposition decision: **01.04.2020**
(45) Mention of the grant of the patent: 14.03.2012
(21) Application number: 10170545.7
(22) Date of filing: 22.07.2010
(51) Int. Cl.: A61M 1/16

(54) **Wearable artificial kidney with regeneration system**
Tragbare künstliche Niere mit Regenerationssystem
Rein artificiel portable avec système de régénération

(30) Priority: 22.07.2009 IT BO20090471; 22.07.2009 IT BO20090472
(43) Date of publication of application: 09.02.2011
(73) Proprietor: Bellco S.r.l., Mirandola (IT)
(72) Inventor: Cianciavicchia, Domenico, 64040 Cavuccio (IT); Ronco, Claudio, 36100 Vicenza (IT)
(74) Representative: Maschio, Antonio

(56) References cited:
- WO-A1-99/42150
- WO-A1-2004/058385
- WO-A1-2010/144189
- WO-A2-2005/044339
- WO-A2-2008/106191
- US-A- 3 579 441
- US-A- 3 669 880
- US-A- 3 926 797
- US-A- 5 284 470
- US-A1- 2003 098 270
- US-A1- 2006 241 543

## Description

The present invention relates to a wearable artificial kidney with a regeneration system.

Dialysis is a blood purification method capable of restoring the hydrosaline balance and eliminating excess water and toxic substances that accumulate in the body during renal failure. In practice, in the application of this method, once the blood has been removed from the patient it flows through the arterial line to enter a dialyser in which the blood is purified, after which it flows out through the venous line to be returned the patient purified.

Haemodialysis is a dialysis technique based on the diffusion through a membrane of substances from a more concentrated solution to a less concentrated solution. In substance, due to the difference in concentration between the blood and the dialysis solution, the phenomenon of diffusion of the substances through the membrane occurs.

In practice, blood is made to flow in the opposite direction to a dialysis solution from opposite sides of an appropriate semi-permeable membrane. Specifically, the dialysis solution does not contain those toxic substances that are to be eliminated from the blood, such as urea, uric acid, creatinine, phosphorus, etc., while it contains a precise quantity of other substances that must be returned to the body, such as sodium, calcium, magnesium, potassium, etc.

Another dialysis technique is haemofiltration, which is based on the concept of performing ultrafiltration (separation of water from blood) creating a pressure gradient through the walls of a highly permeable membrane.

In this way, an ultrafiltrate that is directly correlated in quantity to the pressure exerted on the blood and to the permeability of the membrane is obtained. The ultrafiltrate will be composed of plasma water in which uremic toxins, which are capable of passing through the membrane, are dissolved. Haemofiltration is therefore based on a purely convective process.

To remove sufficient quantities of toxic substances from the blood large quantities of ultrafiltrate must be obtained. All this would cause an excessive dehydrataion of the body and serious depletion of essential substances (such as sodium, calcium, buffers etc.), unless a solution with suitable composition and volume adapted to balance the ultrafiltration flow is reinfused into the blood.

Finally, a further dialysis technique is haemodiafiltration, in which blood is purified using a combination of the phenomena of diffusion and convection.

One of the problems that patients subjected to dialysis must face is that of having to go to equipped structures, such as hospitals, to receive treatment. In order to evaluate the problem correctly, it must be specified that many patients require 3 or 4 sessions a week. As can easily be understood, this obligation can considerably compromise the patient's quality of life.

To overcome this problem, it has recently been recognized that there is a need to develop portable and/or wearable artificial kidneys, which, if worn every day for several hours a day, can ensure that patients have a satisfactory quality of life despite the fact that they require dialysis treatment. The present invention falls within this context.

Generally, portable and/or wearable artificial kidneys comprise a regeneration system to allow re-use of the dialysis solution delivered from the filter or re-use of the ultrafiltrate as reinfusion solution. The regeneration system used to date is based purely on selective adsorption techniques, which on the whole have given relatively unsatisfactory results to allow true efficiency and, therefore, widespread use of portable and/or wearable artificial kidneys. Devices of this kind are disclosed in US-2006/241543 and US-3579441.

The object of the present invention is to produce a wearable artificial kidney with technical features capable of ensuring efficient regeneration of the dialysis solution, or effective regeneration of the ultrafiltrate, thereby optimizing the efficiency of the portable and/or wearable artificial kidney.

The subject matter of the present invention is a dialysis circuit of a wearable artificial kidney that is lightweight, has small dimensions and can be self-managed by the patient, and which can also be controlled remotely by means of the GSM GPRS network, the essential features of which are set forth in claims 1 or 2.

For a better understanding of the invention, embodiments are provided below purely by way of non-limiting example, with the aid of the accompanying drawing, wherein:
- Fig. 1 is a schematic view of a haemodialysis circuit of a wearable artificial kidney given as an example,
- Fig. 2 is a schematic view of a haemodialysis circuit of a wearable artificial kidney according to the present invention, and
- Fig. 3 is a schematic view of a haemofiltration circuit of a wearable artificial kidney given as an example and
- Fig. 4 is a schematic view of a haemofiltration circuit of a wearable artificial kidney given as an example.

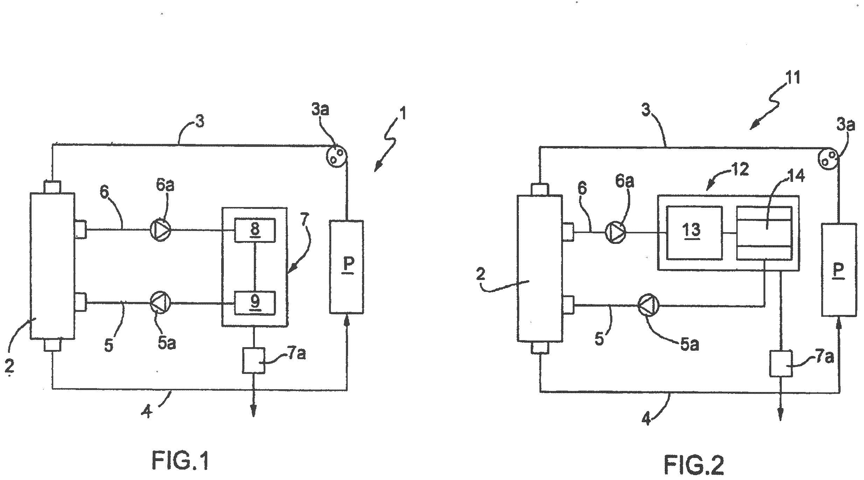

In Fig. 1 the numeral 1 indicates as a whole a first preferred embodiment of a dialysis circuit of a wearable artificial kidney given as example.

The dialysis circuit 1 comprises a haemodialysis filter 2 (known and not described in detail), an arterial line 3 which is responsible for transporting blood from a patient P to the filter 2, a pump 3a attached to the arterial line 3 to guarantee movement of blood, a venous line 4 which is responsible for transporting blood from the filter 2 to the patient P, an inlet line 5 and an outlet line 6 respectively to feed dialysis solution into and to remove dialysis solution from the filter 2, a pair of pumps 5a and 6a attached respectively to the inlet line 5 and to the outlet line 6 of the dialysis solution, a purification unit 7 adapted to receive the dialysis solution from the outlet line 6 and to feed it, purified and appropriately regenerated, into the inlet line 5 so that it can be re-used in the filter 2.

The purification unit 7 comprises a filtration device 8, selected from a nanofiltration, microfiltration or reverse osmosis device, and a regeneration device 9 adapted to provide the dialysis solution with an appropriate concentration of those salts required for dialysis treatment, such as sodium, calcium, magnesium, potassium, etc., and retained by the filtration device 8.

In this way, the dialysis solution used for dialysis is reproduced from the dialysis solution delivered from the filter 2.

A solution of this type offers the considerable advantage of performing efficient purification and regeneration of the dialysis solution so that it can be re-used as such, thereby ensuring highly efficient haemodialysis treatment by means of a wearable artificial kidney.

The purification unit 7 also comprises a device 7a for elimination of plasma water and for checking the patient's weight reduction.

In Fig. 2 the numeral 11 indicates as a whole a dialysis circuit of a wearable artificial kidney according to the present invention.

The parts of the dialysis circuit 11 identical to those of the dialysis circuit 1 will be indicated with the same numerals and will not be described for a second time.

The dialysis circuit 11 comprises a purification unit 12, in turn comprising a microfiltration or nanofiltration device 13 and an adsorption device 14.

In fact, the microfiltration or nanofiltration device 13, on the basis of the cut-off of the membranes used, can retain all or part of the toxic substances and the dissolved salts (sodium, potassium, calcium, etc.) present in plasma water.

To recapitulate, if the filtration device is capable of also stopping salts, a system for reintegration of these salts is required; however, if the filtration device is only capable of stopping part of the toxic molecules, adsorption devices must be used to remove toxins not stopped by the filters.

The solution described above also allows efficient regeneration of the dialysis solution, thereby guaranteeing effective dialysis treatment of the wearable artificial kidney.

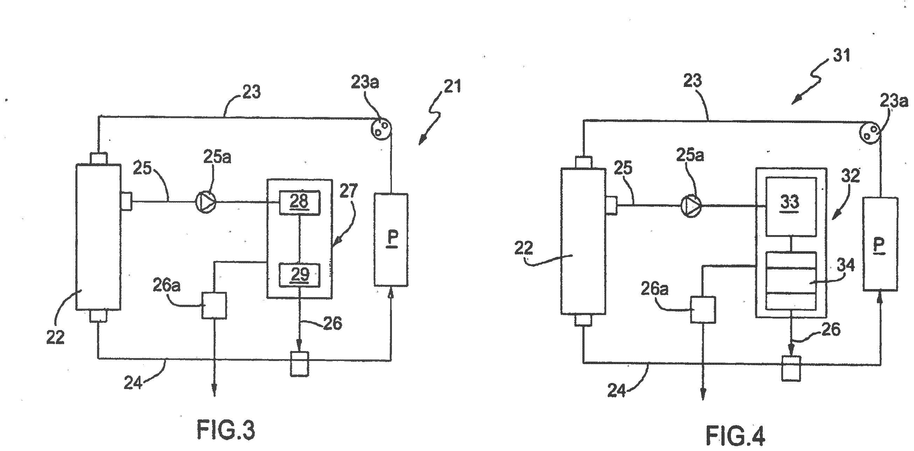

In Fig. 3 the numeral 21 indicates as a whole an embodiment of a haemofiltration circuit of a wearable artificial kidney given as an example.

The haemofiltration circuit 21 comprises a haemofilter 22 (known and not described in detail), an arterial line 23, which is responsible for transporting blood from a patient P to the haemofilter 22, a pump 23a attached to the arterial line 23 to guarantee movement of blood, a venous line 24 which is responsible for transporting blood from the haemofilter 22 to the patient P, an ultrafiltrate line 25, a pump 5a attached to the ultrafiltrate line 25, a reinfusion line 26 and a purification unit 27 adapted to receive plasma water from the ultrafiltrate line 25 and to feed it, purified and appropriately regenerated, into the reinfusion line 26 so that it can be reinfused into the blood through the venous line 24.

The purification unit 27 comprises a filtration device 28, selected from a nanofiltration, microfiltration or reverse osmosis device, and a regeneration device 29 adapted to provide plasma water with an appropriate concentration of those salts retained by the filtration device 28 but necessary for correct ion composition of the blood.

In this way, the plasma water useful for reinfusion in haemofiltration is regenerated. A solution of this kind offers the considerable advantage of performing efficient purification and regeneration of plasma water so that it can be re-used as reinfusion solution, thereby guaranteeing highly efficient haemofiltration treatment by means of the wearable artificial kidney.

The purification unit 27 comprises a device 26a for elimination of plasma water and for checking the patient's weight reduction.

In Fig. 4 the numeral 31 indicates as a whole a haemofiltration circuit of a wearable artificial kidney.

The parts of the haemofiltration circuit 31 identical to those of the haemofiltration circuit 21 will be indicated with the same numerals and will not be described for a second time.

The haemofiltration circuit 31 comprises a purification unit 32, in turn comprising a microfiltration or nanofiltration device 33 and an adsorption device 34. In fact, the microfiltration or nanofiltration device 33, on the basis of the cut-off of the membranes used, can retain all or part of the toxic substances and the dissolved salts (sodium, potassium, calcium, etc.) present in plasma water.

To recapitulate, if the filtration device is capable of also stopping salts, a system for reintegration of these salts is required; however, if the filtration device is only capable of stopping part of the toxic molecules, adsorption devices must be used to remove the toxins not stopped by the filters.

The solution described here also allows efficient regeneration of plasma water as reinfusion solution, thereby guaranteeing effective haemofiltration treatment of the portable artificial kidney.

## Claims

1. A dialysis circuit (1; 11) of a wearable artificial kidney comprising a dialysis filter (2), an inlet line (5) to feed a dialysis solution into said filter (2), an outlet line (6) to remove the dialysis solution from said filter (2) connected to said inlet line (5), an arterial line (3) which is responsible for transporting blood from a patient (P) to the filter (2), a venous line (4) which is responsible for transporting blood from the filter (2) to the patient (P), a plurality of pumps (3a, 5a, 6a) adapted for circulation both of the blood and of the dialysis solution and a purification unit (7) adapted to perform filtration of the dialysis solution coming from said outlet line (6) and directed toward said inlet line (5); said dialysis circuit being **characterized in that** said purification unit (7) comprises a filtration device (8) included in the group composed of a nanofiltration filtration device or a microfiltration filtration device, and adsorption means (14; 34) arranged downstream of said filtration device (13; 33) and adapted to retain the molecules not stopped by said filtration device (13; 33); the plurality of pumps (3a, 5a, 6a) comprise a first pump (3a) attached to the arterial line (3) to guarantee movement of blood and a pair of pumps (5a, 6a) attached respectively to the inlet line (5) and to the outlet line (6) of the dialysis solution.

2. A haemofiltration circuit (21; 31) of a wearable artificial kidney comprising a haemofilter (22), an arterial line (23) which is responsible for transporting blood from a patient (P) to the haemofilter (22), a venous line (24) which is responsible for transporting blood from the haemofilter (2) to the patient (P), an ultrafiltrate line (25) extending from the haemofilter, a reinfusion line (26) adapted to reinfuse purified plasma water into said arterial line (23) and/or into said venous line (24) and connected to said ultrafiltrate line (25), a plurality of pumps (23a, 25a) adapted for circulation both of blood and of plasma water and a purification unit (27) adapted to perform filtration of the plasma water coming from said ultrafiltrate line (25) and directed toward said reinfusion line (26); said haemofiltration circuit being **characterized in that** said purification unit (27) comprises a filtration device (28) included in the group composed of a nanofiltration filtration device or a microfiltration filtration device, and adsorption means (14; 34) arranged downstream of said filtration device (13; 33) and adapted to retain the molecules not stopped by said filtration device (13; 33); the purification unit comprising a device (26a) for elimination of plasma water and for checking the patient's weight reduction.

3. A wearable artificial kidney **characterized in that** it comprises a dialysis circuit according to claim 1 or 2.

## Patentansprüche

1. Dialysekreislauf (1; 11) einertragbaren künstlichen Niere, umfassend einen Dialysefilter (2), eine Einlassleitung (5), um eine Dialyselösung in den Filter (2) zuzuführen, und eine Auslassleitung (6), um die Dialyselösung aus dem mit der Einlassleitung (5) verbundenen Filter (2) zu entfernen, eine arterielle Leitung (3), die für ein Transportieren von Blut von einem Patienten (P) zu dem Filter (2) verantwortlich ist, eine venöse Leitung (4), die zum Transportieren von Blut von dem Filter (2) zu dem Patienten (P) verantwortlich ist, mehrere Pumpen (3a, 5a, 6a), die für die Zirkulation sowohl des Blutes als auch der Dialyselösung geeignet sind, und eine Reinigungseinheit (7), die geeignet ist, um eine Filtration der Dialyselösung auszuführen, die von der Auslassleitung (6) kommt und in Richtung der Einlassleitung (5) gelenkt wird; wobei der Dialysekreislauf **dadurch gekennzeichnet ist, dass** die Reinigungseinheit (7) eine Filtrationsvorrichtung (8), die in der Gruppe beinhaltet ist, die aus einer Nanofiltrationsfiltrationsvorrichtung oder einer Mikrofiltrationsfiltrationsvorrichtung besteht, und Adsorptionsmittel (14; 34) umfasst, die stromabwärts von der Filtrationsvorrichtung (13; 33) angeordnet und geeignet sind, um die von der Filtrationsvorrichtung (13; 33) nicht gestoppten Moleküle zurückzuhalten; wobei die mehreren Pumpen (3a, 5a, 6a) eine erste Pumpe (3a), die an der arteriellen Leitung (3) befestigt ist, um eine Bewegung von Blut zu gewährleisten, und ein Paar von Pumpen (5a, 6a) umfassen, die an der Einlassleitung (5) beziehungsweise der Auslassleitung (6) für die Dialyselösung befestigt sind.

2. Hämofiltrationskreislauf (21; 31) einer tragbaren künstlichen Niere, umfassend einen Hämofilter (22), eine arterielle Leitung (23), die für das Transportieren von Blut von einem Patienten (P) zu dem Hämofilter (22) verantwortlich ist, eine venöse Leitung (24), die für das Transportieren von Blut von dem Hämofilter (2) zu dem Patienten (P) verantwortlich ist, eine Ultrafiltratleitung (25), die sich von dem Hämofilter aus erstreckt, eine Reinfusionsleitung (26), die geeignet ist, gereinigtes Plasmawasser in die arterielle Leitung (23) und/oder in die venöse Leitung (24) zu reinfundieren und die mit der Ultrafiltratleitung (25) verbunden ist, mehrere Pumpen (23a, 25a), die für die Zirkulation sowohl von Blut als auch des Plasmawassers geeignet sind, und eine Reinigungseinheit (27), die geeignet ist, um eine Filtration des Plasmawassers durchzuführen, das aus der Ultrafilterleitung (25) kommt und in Richtung der Reinfusionsleitung (26) gelenkt wird; wobei der Hämofiltrationskreislauf **dadurch gekennzeichnet ist, dass** die Reinigungseinheit (27) eine Filtrationsvorrichtung (28), die in der Gruppe enthalten ist, die aus einer Nanofiltrationsfiltrationsvorrichtung oder einer Mikrofiltrationsfiltrationsvorrichtung besteht, und Adsorptionsmittel (14; 34) umfasst, die stromabwärts von der Filtrationsvorrichtung (13; 33) angeordnet und geeignet sind, um die von der Filtrationsvorrichtung (13; 33) nicht gestoppten Moleküle zurückzuhalten; wobei die Reinigungseinheit eine Vorrichtung (26a) für eine Beseitigung von Plasmawasser und zum Überprüfen der Gewichtsreduktion des Patienten umfasst.

3. Tragbare künstliche Niere, **dadurch gekennzeichnet, dass** sie einen Dialysekreislauf nach Anspruch 1 oder 2 umfasst.

## Revendications

1. Circuit de dialyse (1 ; 11) d'un rein artificiel portatif comprenant un filtre de dialyse (2), une ligne d'entrée (5) pour introduire une solution de dialyse dans ledit filtre (2), une ligne de sortie (6) pour ôter la solution de dialyse à partir dudit filtre (2) relié à ladite ligne d'entrée (5), une ligne artérielle (3) permettant de transporter le sang d'un patient (P) vers le filtre (2), une ligne veineuse (4) permettant de transporter le sang du filtre (2) au patient (P), une pluralité de pompes (3a, Sa, 6a) adaptées pour la circulation à la fois du sang et de la solution de dialyse, et une unité de purification (7) adaptée pour effectuer la filtration de la solution de dialyse provenant de ladite ligne de sortie (6) et dirigée vers ladite ligne d'entrée (5) ; ledit circuit de dialyse étant **caractérisé en ce que** ladite unité de purification (7) comprend un dispositif de filtration (8) inclus dans le groupe composé d'un dispositif de filtration par nanofiltration ou d'un dispositif de filtration par microfiltration, et des moyens d'adsorption (14 ; 34) disposés en aval dudit dispositif de filtration (13 ; 33) et adaptés pour retenir les molécules non arrêtées par ledit dispositif de filtration (13 ; 33) ; la pluralité de pompes (3a, Sa, 6a) comprend une première pompe (3a) fixée à la ligne artérielle (3) pour garantir le mouvement du sang et une paire de pompes (Sa, 6a) fixées respectivement à la ligne d'entrée (5) et à la ligne de sortie (6) de la solution de dialyse.

2. Circuit d'hémofiltration (21 ; 31) d'un rein artificiel portatif comprenant un hémofiltre (22), une ligne artérielle (23) permettant de transporter le sang d'un patient (P) à l'hémofiltre (22), une ligne veineuse (24) permettant de transporter le sang de l'hémofiltre (2) au patient (P), une ligne d'ultrafiltrat (25) s'étendant de l'hémofiltre, une ligne de réinfusion (26) adaptée pour réinfuser de l'eau plasmatique purifiée dans ladite ligne artérielle (23) et/ou dans ladite ligne veineuse (24) et reliée à ladite ligne d'ultrafiltrat (25), une pluralité de pompes (23a, 25a) adaptées pour la circulation à la fois de sang et d'eau plasmatique, et une unité de purification (27) adaptée pour effectuer une filtration de l'eau plasmatique provenant de ladite ligne d'ultrafiltrat (25) et dirigée vers ladite ligne de réinfusion (26) ; ledit circuit d'hémofiltration étant **caractérisé en ce que** ladite unité de purification (27) comprend un dispositif de filtration (28) inclus dans le groupe composé d'un dispositif de filtration par nanofiltration ou d'un dispositif de filtration-microfiltration, et des moyens d'adsorption (14 ; 34) disposés en aval dudit dispositif de filtration (13 ; 33) et adaptés pour retenir les molécules non arrêtées par ledit dispositif de filtration (13 ; 33) ; l'unité de purification comprenant un dispositif (26a) pour ôter l'eau plasmatique et pour vérifier la réduction de poids du patient.

3. Rein artificiel portatif, **caractérisé en ce qu'**il comprend un circuit de dialyse selon la revendication 1 ou 2.
